# EUROPÄISCHE PATENTANMELDUNG

(11) **EP 1 762 627 A1**
(43) Veröffentlichungstag der Anmeldung: **14.03.2007**
(21) Anmeldenummer: 05019665.8
(22) Anmeldetag: 09.09.2005
(51) Int. Cl.: C12Q 1/68

(54) **Verfahren zur Aktivierung einer Nukleinsäure für eine Polymerase-Reaktion**

(71) Anmelder: QIAGEN GmbH, 40724 Hilden (DE)
(72) Erfinder: Korfhage, Christian, 40764 Langenfeld (DE); Löffert, Dirk, 40589 Düsseldorf (DE)
(74) Vertreter: Leidescher, Thomas

(57) **Zusammenfassung**

Die vorliegende Erfindung betrifft ein Verfahren zur Aktivierung einer Nukleinsäure für eine Strand-Displacement-Reaktion mit den Schritten: (a) Erwärmen einer Nukleinsäure (vorzugsweise einer doppelsträngigen DNA) auf eine Temperatur von 50 bis 90°C, (b) Abkühlen der Nukleinsäure auf eine Temperatur, bei der eine Polymerase keine wesentliche Abnahme der Aktivität zeigt, und (c) Starten der Strand-Displacement-Reaktion durch Zugabe einer hitzelabilen Polymerase zu der Nukleinsäure. Bei einem alternativen Verfahren, bei dem eine hitzestabile Polymerase eingesetzt wird, kann die Polymerase gleich in Schritt (a) zugegeben werden. Das Verfahren kann sowohl mit gereinigter Nukleinsäure als auch in einer Zelle enthaltener Nukleinsäure durchgeführt werden.

SEQUENCE LISTING

<110> QIAGEN GmbH

<120> Verfahren zur Aktivierung einer Nukleinsäure für eine
Polymerase-Reaktion

<130> QIA 11875P-EP

<160> 10

<170> PatentIn version 3.3

<210> 1
<211> 19
<212> DNA
<213> Homo sapiens

<400> 1
tctttccact ccattgcat 19

<210> 2
<211> 18
<212> DNA
<213> Homo sapiens

<400> 2
ggaatggaat caacccaa 18

<210> 3
<211> 20
<212> DNA
<213> Homo sapiens

<400> 3
gtctcaagtc agtgtacagg 20

<210> 4
<211> 20
<212> DNA
<213> Homo sapiens

<400> 4
gtgatagcat tgctttcgtg 20

<210> 5
<211> 30
<212> DNA
<213> Homo sapiens

<400> 5
tgatggcatt actggcactt tgagttttac 30

<210> 6
<211> 22
<212> DNA
<213> Homo sapiens

<400> 6
gtctttagct gctgaggaaa tg 22

<210> 7

<211>  21
<212>  DNA
<213>  Homo sapiens

<400>  7
agcagaattc tgcacatgac g                                                21


<210>  8
<211>  24
<212>  DNA
<213>  Homo sapiens

<400>  8
[?]gaactgctc cttggcaggg attt                                           24


<210>  9
<211>  18
<212>  DNA
<213>  Homo sapiens

<400>  9
tgctccctgt cccatctg                                                    18


<210>  10
<211>  24
<212>  DNA
<213>  Homo sapiens

<400>  10
agacagtatg cctttatttc accc                                             24

## Beschreibung

Die vorliegende Erfindung betrifft ein Verfahren zur Aktivierung einer Nukleinsäure, insbesondere einer Desoxyribonukleinsäure (DNA), für eine Polymerase-Reaktion, insbesondere eine Strand-Displacement-Reaktion (Strangverdrängungsreaktion).

Unter einer Polymerase-Reaktion im Sinne der Erfindung wird die Polymerase-Aktivität einer Nukleinsäure-Polymerase verstanden, also das Polymerisieren von Nukleotiden an ein freies 3'-OH-Ende wobei der komplementäre Strang als Template dient. Dabei kann ein eventuell 3' von dem freien 3'-OH-Ende liegender Strang entweder verdrängt werden kann (Strand-Displacement-Reaktion, siehe unten) oder dieser durch eine 5'-3'-Exonuklease-Aktivität der Polymerase auch abgebaut und durch den neu synthetisierten Strang ersetzt werden kann (beispielsweise in einer Nick-Displacement-Reaktion).

Eine Strand-Displacement-Reaktion (SDR) ist eine Methode, bei der eine Polymerase-Reaktion mit Oligonukleotiden gestartet wird, wobei während der Reaktion ein Abschälen eines "alten" Stranges ("Strand-Displacement") einer doppelsträngigen Nukleinsäure von dem anderen "alten" (komplementären) Strang bewirkt wird, um ein Binden von Oligonukleotiden an den anderen "alten" Strang zu ermöglichen. Wichtig für die Reaktion ist die Initiierung, wobei man verschiedene Techniken unterscheiden kann:
(A) Eine Trennung der beiden hybridisierenden Nukleinsäure-Stränge (z.B. DNA-Stränge) kann durch eine Hitzedenaturierung bei 95°C erfolgen. Bei dieser Temperatur werden nachweislich die beiden DNA-Stränge voneinander getrennt, so dass Oligonukleotide an den denaturierten (d.h. voneinander getrennten) DNA-Strängen binden können. Die Initiation der SDR kann dann erfolgen (z.B. Protokoll des GenomiPhi-Kits, Amersham Biosciences GmbH, Freiburg i. Br., Deutschland). Dieses Verfahren hat jedoch einen wesentlichen Nachteil: Eine Erhitzung auf 95°C führt zu einer Schädigung der DNA, z.B. durch Depurinisierung oder Strangbrüche (Suzuki T., Ohsumi S., Makino, K. (1994), Mechanistic studies on depurination and apurinic site chain breakage in oligodeoxyribonucleotides, Nucleic Acid Res. 22(23): 4997-5003).
(B) Eine Trennung der beiden hybridisierenden Nukleinsäure-Stränge (DNA-Stränge) kann auch durch eine Alkali-Denaturierung (Protokoll des REPLI-g Kits, QIAGEN GmbH, Hilden, Deutschland) erfolgen. Dies hat jedoch den Nachteil, dass nach der Alkali-Zugabe neutralisiert werden muss. Dies bedeutet zusätzliche Pipettierschritte und eine Veränderung im Reaktionsmilieu.
(C) Eine nächste Methode versucht keine Strangtrennung zu erreichen, sondern verwendet Endonukleasen, um Einzelstrangbrüche einzufügen, an deren 3'-OH Ende die Polymerase-Reaktion starten kann (vergl. z.B. US 6,884,586).
(D) Eine vierte Methode schließlich verwendet keine Denaturierung, wie z.B. von Notomi T., Okayama H., Masubuchi H., Yonekawa T., Watanabe K., Amino N., Hase T. (2000), Loop-mediated isothermal amplification of DNA, Nucleic Acids Res. 15; 28(12):E63 beschrieben. Dies führt jedoch zu deutlich schlechteren Ergebnissen, da vermutlich die in der DNA enthalten Einzelstrangbrüche für die Verlängerung in einer SDR verwendet werden.

Aufgabe der vorliegenden Erfindung ist es daher, ein Verfahren zur Aktivierung einer Nukleinsäure, insbesondere einer Desoxyribonukleinsäure (DNA), für eine Strand-Displacement-Reaktion (Strangverdrängungsreaktion) anzugeben, das die oben beschriebenen Nachteile des Standes der Technik nicht aufweist. Diese Aufgabe löst die Erfindung durch das Verfahren gemäß der unabhängigen Ansprüche 1 und 2. Weitere vorteilhafte Ausgestaltungen der vorliegenden Erfindung sind in den abhängigen Ansprüchen, der Beschreibung, den Beispielen und der Zeichnung angegeben.

Nachfolgend werden einige der verwendeten Begriffe näher erläutert.

Strand-Displacement-Reaktion (SDR): Unter Strand-Displacement-Reaktion wird hier jede Reaktion verstanden, bei der eine Polymerase verwendet wird, die eine Strand-Displacement Aktivität aufweist, oder bei der eine Reaktionsbedingung verwendet wird, die ein Strand-Displacement ermöglicht. Hierzu zählen z.B. die Strand-Displacement-Amplification (SDA) genauso wie die Multiple-Displacement-Amplification (MDA) oder die Rolling-Circle-Amplification (RCA) sowie alle Unterformen dieser Reaktionen, wie z.B. Restriction-aided RCA (RCA-RCA) oder MDA mit *nested* Primern, lineare und exponentielle Strand-Displacement-Reaktionen oder auch Helicase-Dependent Amplification (vergl. z.B. die europäischen Patentanmeldungen Nr. 20050112639, 20050074804, 20050069939 und 20050069938, sowie Wang G., Maher E., Brennan C., Chin L., Leo C., Kaur M., Zhu P., Rook M., Wolfe J.L., Makrigiorgos G.M. (2004), DNA amplification method tolerant to sample degradation, Genome Res. Nov;14(11):2357-2366; Milla M.A., Spears P.A., Pearson R.E., Walker G.T. (1998), Use of the restriction enzyme AvaI and exo-Bst polymerase in strand displacement amplification, Biotechniques Mar;24(3):392-396; Nagamine K., Watanabe K., Ohtsuka K., Hase T., Notomi T. (2001), Loop-mediated isothermal amplification reaction using a nondenatured template, Clin Chem. 47(9):1742-1743; Notomi et al 2001 (siehe oben); Lage J.M., Leamon J.H., Pejovic T., Hamann S., Lacey M., Dillon D., Segraves R., Vossbrinck B., Gonzalez A., Pinkel D., Albertson D.G., Costa J., Lizardi P.M. (2003), Whole genome analysis of genetic alterations in small DNA samples using hyperbranched strand displacement amplification and array-CGH, Genome Res.13(2):294-307; und Vincent M., Xu Y., Kong H. (2004), Helicase-dependent isothermal DNA amplification, EMBO Rep. 5(8):795-800).

Strand-Displacement-Polymerase: Zu den Strand-Displacement-Polymerasen gehören alle Polymerasen, die ein Strand-Displacement durchführen können. Hierzu zählen Enzyme, wie z.B. Phi29-DNA-Polymerase, Klenow DNA-Polymerase, Vent DNA Polymerase, Deep Vent DNA Polymerase, Bst DNA Polymerase, 9oNm™ DNA Polymerase und Bca DNA Polymerase. Die Strand-Dipslacement-Polymerasen können auch in mutierter Form vorliegen, z.B. als sogenannte exominus-Varianten (d.h. ohne Exonuklease Aktivität).

DNA: Desoxyribonukleinsäure (DNA) kommt natürlicherweise in Organismen vor, kann aber auch außerhalb von Organismen vorkommen oder diesem hinzugefügt worden sein. Die Länge der DNA kann unterschiedlich sein. Die DNA kann durch Veränderungen modifiziert sein. Die Basen der DNA können modifiziert sein. Die DNA kann Basenanaloga (z.B. auch non-Purin oder non-Pyrimidin Analoga) oder Nukleotidanaloga (z.B. PNA) enthalten. Die DNA kann Anhänge enthalten, wie z.B. Proteine oder Aminosäuren.

Die vorliegende Erfindung betrifft somit ein Verfahren zur Aktivierung einer Nukleinsäure (insbesondere einer doppelsträngigen DNA) für eine Strand-Displacement-Reaktion, wobei das Verfahren die folgenden Schritte umfaßt: (a) Erwärmen der Nukleinsäure auf eine (im Vergleich zu herkömmlichen Verfahren moderate) Temperatur von 50 bis 90°C; (b) Abkühlen der Nukleinsäure auf eine Temperatur, bei der eine Polymerase keine wesentliche Abnahme der Aktivität zeigt; und (c) Starten der Strand-Displacement-Reaktion durch Zugabe einer Polymerase zu der Nukleinsäure. Die bei einem bisher verwendeten Verfahren eingesetzte hohe Temperatur von 95°C, die sich, wie oben beschrieben, insofern negativ auswirkt, als eine eingesetzte Nukleinsäure (z.B. DNA) erheblich durch Strangbrüche und Depurinisierung) geschädigt wird, kann durch das erfindungsgemäße Verfahren vermieden werden. Auch auf die bisher häufig angewandte Trennung doppelsträngiger Nukleinsäuren mittels Alkalibehandlung mit den entsprechenden nachteiligen Begleitumständen kann durch das neue Verfahren verzichtet werden. Das erfindungsgemäße Verfahren bietet somit eine die eingesetzte Nukleinsäure schonende Möglichkeit, eine Strand-Displacement-Reaktion vorzubereiten und durchzuführen.

Die oben genannte Variante wird bevorzugt dann eingesetzt, wenn eine hitzelabile Polymerase zum Einsatz kommt. Wird dagegen eine hitzestabile Polymerase verwendet, die eine zumindest kurzfristige Erwärmung auf bis zu 90°C, bevorzugt auf bis zu 80°C, besonders bevorzugt auf bis zu 70°C und insbesondere auf bis zu 65°C ohne nennenswerte Einbuße ihrer Aktivität übersteht, kann diese bereits dem Schritt (a) zugesetzt werden, und eine Abkühlung des Reaktionsansatzes vor der Polymerasezugabe kann unterbleiben. Das alternative Verfahren umfaßt daher den Schritt (a) Erwärmen der Nukleinsäure zusammen mit einer hitzestabilen Polymerase auf eine (im Vergleich zu herkömmlichen Verfahren moderate) Temperatur von 50 bis 90°C.

Die moderate Temperatur, bei der Zellen oder isolierte DNA erhitzt werden, liegt zwischen 50°C und 90°C, besonders bevorzugt bei 55-80°C, ganz besonders bevorzugt bei 60-70°C und insbesondere bei 65°C. Die Erhitzung der DNA oder der Zellen kann beispielsweise direkt im SDR-Reaktionsgemisch erfolgen.

Die Erfindung beschreibt somit eine Aktivierung einer Nukleinsäure (insbesondere DNA) für eine Strand-Displacement Reaktion durch einen moderaten Erhitzungsschritt. Die erfindungsgemäße Methode umfaßt bei Verwendung von isolierter Nukleinsäure (DNA) und einer hitzelabilen Strand-Displacement-Polymerase dementsprechend folgende Teilschritte: (1) Die Nukleinsäure (DNA) wird auf eine moderat hohe Temperatur erhitzt. (2) Die Nukleinsäure (DNA) wird abgekühlt, wobei die Temperatur nach dem Abkühlvorgang maximal eine Höhe haben darf, bei der die Polymerase noch nicht deutlich an Aktivität verliert. Die Nukleinsäure wird bevorzugt auf eine Temperatur von 4°C bis 45°C abgekühlt, besonders bevorzugt auf einen Bereich von 15°C bis 42°C und ganz besonders bevorzugt auf einen Bereich von 25°C und 37°C. (3) Die SDR-Reaktion wird durch Zugabe der (hitzelabilen) Polymerase gestartet.

Die oben vorgestellte Variante wird bevorzugt dann eingesetzt, wenn eine hitzelabile Polymerase verwendet wird. Wird dagegen eine hitzestabile Polymerase eingesetzt, kann diese bereits dem Schritt (1) zugegeben werden.

Das erfindungsgemäße Verfahren kann aber nicht nur für reine bzw. gereinigte DNA verwendet werden, sondern auch für DNA, die noch in einem Zellverbund enthalten ist. Die Methode für die Verwendung von DNA, die noch im Zellverbund enthalten ist, und einer (vorzugsweise hitzelabilen) Strand-Displacement-Polymerase hat dementsprechend folgende Teilschritte: (1) Die DNA-haltigen Zellen werden auf eine moderat hohe Temperatur erhitzt. (2) Die DNA-haltigen Zellen werden abgekühlt, wobei die Temperatur nach dem Abkühlvorgang maximal eine Höhe haben darf, bei der die Polymerase noch nicht deutlich an Aktivität verliert. (3) Die SDR-Reaktion wird durch Zugabe der Polymerase gestartet.

Bei beiden Methoden kann auch die hitzelabile Strand-Displacement-Polymerase auch durch eine hitzestabile Strand-Displacement-Polymerase ersetzt werden. Dann kann der Teilschritt (1) jeweils direkt mit der Polymerase durchgeführt werden.

In der Zeichnung zeigen
- Fig. 1: die Ausbeute der Reaktionen aus Beispiel 1;
- Fig. 2: die Ct-Werte der Real-time PCR aus Beispiel 1;
- Fig. 3: die Ausbeute der Reaktionen aus Beispiel 2;
- Fig. 4: die Ct-Werte der Real-time PCR aus Beispiel 2;
- Fig. 5: die Ct-Werte der Real-time PCR aus Beispiel 3;
- Fig. 6: die Ausbeute der Reaktionen aus Beispiel 4; und
- Fig. 7: die Ct-Werte der Real-time PCR aus Beispiel 4.

Die Erfindung wird nachfolgend anhand von Beispielen näher erläutert.

### Beispiel 1

Das Beispiel soll zeigen, dass durch einen einfachen Temperaturaktivierungsschritt eine SDR (hier eine Multiple-Displacement-Amplification, MDA) ausgehend von Vollblut ermöglicht wird, die hinsichtlich der DNA-Ausbeute und DNA-Qualität vergleichbar zu einer Reaktion nach dem Stand der Technik (Kontrollreaktion) ist.

Erfindungsgemäße Reaktion: Die MDA-Reaktion wurde mit den REPLI-G Reagenzien (QIAGEN GmbH, Hilden, Deutschland) durchgeführt. Jeweils 0,5, 1 und 2 µl Vollblut, stabilisiert durch EDTA oder Citrat, wurden mit 12,5 µl 4x Reaction Mix (dieser enthält Oligonukleotide mit einer Zufallssequenz, Reaktionspuffer und dNTPs) und Wasser auf ein Volumen von 39,5 µl aufgefüllt und anschließend auf 65°C erwärmt. Nach Abkühlen auf Raumtemperatur (ca. 20-25°C) wurde die DNA-Polymerase aus dem REPLI-G Kit zugegeben. Die Reaktion wurde dann für 6 h bei 30°C durchgeführt.

Kontroll-Reaktion nach dem Stand der Technik: Die MDA-Reaktion wurde mit den REPLI-G Reagenzien (QIAGEN) durchgeführt: 0,5 µl Vollblut, stabilisiert durch EDTA, wurden mit 2,5 µl phosphatgepufferter Salzlösung (PBS, aus dem REPLI-G Kit von QIAGEN) versetzt. Anschließend wurden 3,5 µl des frisch angesetzten Denaturierungspuffers (360 mM KOH, 9 mM EDTA, 100 mM DTT) hinzu gegeben und für 10 min auf Eis inkubiert. Das Gemisch wurde nach der Inkubation mit Solution B (REPLI-G Kit) neutralisiert. Das Gemisch wurde mit 12,5 µl 4x Reaction Mix aus dem REPLI-G Kit (dieser enthält Oligonukleotide mit einer Zufallssequenz, Reaktionspuffer und dNTPs) und dest. Wasser auf ein Volumen von 49,5 µl aufgefüllt. Daraufhin wurde 0,5 µl der DNA-Polymerase aus dem REPLI-G Kit zugegeben. Die Reaktion wurde dann für 6 h bei 30°C durchgeführt.

Nach Ablauf der Reaktion wurde die DNA-Konzentration mit PicoGreen nach Hersteller-Protokoll (Molecular Probes Inc., Eugene, Oregon, USA) gemessen. 10 ng der MDA-DNA wurde für eine Real-time PCR (Polymerasekettenreaktion) eingesetzt. 4 verschiedene Loci wurden auf ihre Repräsentanz im Amplifikat hin untersucht:
(a) eine Sequenz, die als Sat bezeichnet wurde
   (Primer-Sequenzen:
   Sat 1.1 TCTTTCCACTCCATTGCAT und
   Satl.2 GGAATGGAATCAACCCAA
(b) eine Sequenz aus dem β-Aktin Gen
   **Primer 1 GTCTCAACTCAGTGTACAGG**
   **Primer 2 GTGATAGCATTGCTTTCGTG** (c) eine Sequenz, die aus dem als "1004" bezeichneten Locus entstammt
   (Probe: TGATGGCATTACTGGCACTTTGAGTTTTAC,
   Primer 1: GTCTTTAGCTGCTGAGGAAATG,
   Primer 2: AGCAGAATTCTGCACATGACG) und
(d) eine Sequenz, die aus dem als "699" bezeichneten Locus entstammt
   (Probe: TGAACTGCTCCTTGGCAGGGATTT,
   Primer 1: TGCTCCCTGTCCCATCTG,
   Primer 2: AGACAGTATGCCTTTATTTCACCC).

Die Real-time PCR Reaktionen wurden im QuantiTect Master Mix (QIAGEN) entsprechend den Protokoll-Anweisungen durchgeführt.

Die Repräsentanz der Loci in der amplifizierten DNA wurde in Ct-Werten (Threshold-Cycles = Ct) gemessen. Der Ct-Wert ist der PCR-Zyklus in der Real-time PCR, an dem das Fluoreszenz-Signal zum ersten Mal meßbar wird. Durch den Ct-Wert kann somit die relative Häufigkeit einer Sequenz in der Probe ermittelt werden. Ist beispielsweise ein Ct-Wert um 1 Zyklus kleiner als in einer Vergleichsprobe, so entspricht dieser Wert einer ca. 2-fach höheren DNA-Ausgangsmenge im Testansatz gegenüber dem Kontrollansatz für die gemessene Sequenz.

Das Ergebnis von Beispiel 1 läßt sich wie folgt zusammenfassen: (1) Die Ausbeute aus den Reaktionen, bei denen die DNA erfindungsgemäß durch einen 65°C-Schritt aktiviert wurde, ist vergleichbar zur Ausbeute der Kontroll-Reaktion. (2) Auch die Repräsentanz der Sequenzen in der amplifizierten DNA ist vergleichbar, wenn 0,5 µl Blut eingesetzt wird. Lediglich die Sequenz des Sat-Locus ist niedriger als in der Kontroll-Reaktion. (3) Größere Volumina als 0,5 µl wirken hemmend auf die MDA Reaktion mit 65°C Aktivierung. Dies ist erkennbar an den höheren Ct-Werten (d.h. schlechtere Repräsentanz der Sequenzen in der amplifizierten DNA).

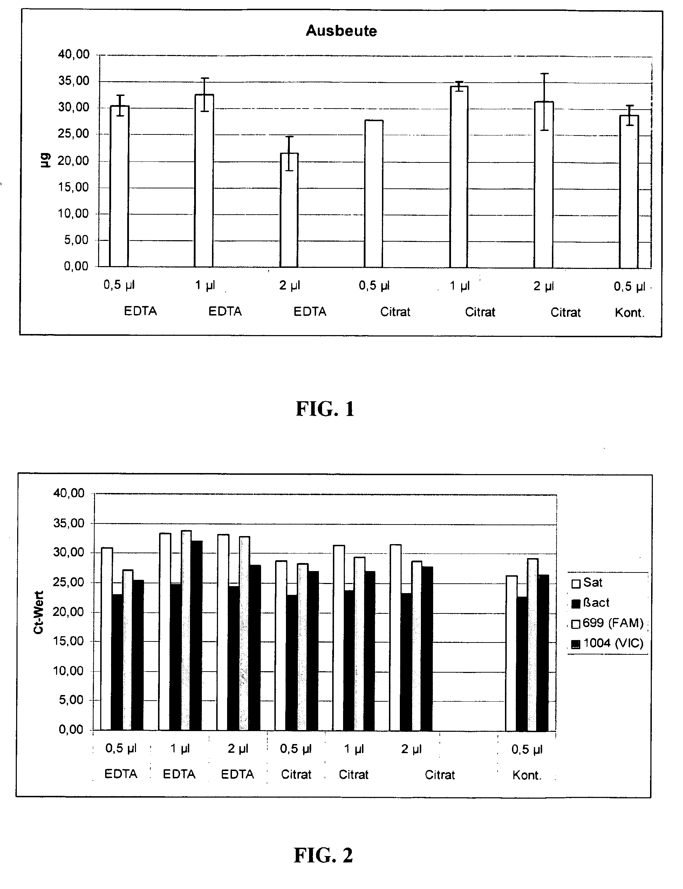

Die Ausbeute der Reaktionen aus Beispiel 1 ist in Fig. 1 graphisch dargestellt. Fig. 2 zeigt die Ct-Werte der Real-time PCR aus Beispiel 1.

### Beispiel 2

Dieses Beispiel dient dazu zu zeigen, dass zu geringe Temperaturen bei einem Temperaturaktivierungsschritt die Qualität der DNA, die während der SDR entsteht, beeinträchtigen kann.

Erfindungsgemäße Reaktion: Die MDA-Reaktion wurde mit den REPLI-G Reagenzien (QIAGEN) durchgeführt: 0,5 µl Vollblut, stabilisiert durch EDTA, wurde mit 12,5 µl 4x Reaction Mix (dieser enthält Oligonukleotide mit einer Zufallssequenz, Reaktionspuffer und dNTPs) und Wasser auf ein Volumen von 39,5 µl aufgefüllt und durch verschiedene Temperaturen aktiviert (30, 40, 45, 50, 55, 60 bzw. 65°C). Nach Abkühlen auf Raumtemperatur (ca. 20-25°C) wurde die DNA-Polymerase aus dem REPLI-G Kit zugegeben. Die Reaktion wurde für 6 h bei 30°C durchgeführt.

Kontroll-Reaktion nach dem Stand der Technik: Die MDA-Reaktion wurde mit den REPLI-G Reagenzien (QIAGEN) durchgeführt: 0,5 µl Vollblut, stabilisiert durch EDTA, wurden mit 2,5 µl PBS (REPLI-G Kit) versetzt. Anschließend wurden 3,5 µl des frisch angesetzten Denaturierungspuffer (360 mM KOH, 9 mM EDTA, 100 mM DTT) hinzu gegeben und für 10 min auf Eis inkubiert. Das Gemisch wurde nach der Inkubation mit Solution B (REPLI-G Kit) neutralisiert. Das Gemisch wurde dann mit 12,5 µl 4x Reaction Mix aus dem REPLI-G Kit (dieser enthält Oligonukleotide mit einer Zufallssequenz, Reaktionspuffer und dNTPs) und dest. Wasser auf ein Volumen von 49,5 µl aufgefüllt. Daraufhin wurde 0,5 µl der DNA-Polymerase aus dem REPLI-G Kit zugegeben. Die Reaktion wurde für 6 h bei 30°C durchgeführt.

Nach Ablauf der Reaktion wurde die DNA-Konzentration mit PicoGreen nach Hersteller-Protokoll (Molecular Probes) gemessen. 10 ng der MDA-DNA wurden für eine Real-time PCR eingesetzt. 2 verschiedene Loci wurden auf ihre Repräsentanz im Amplifikat hin untersucht:
(a) eine Sequenz, die aus dem als "1004" bezeichneten Locus entstammt
   (Probe: TGATGGCATTACTGGCACTTTGAGTTTTAC,
   Primer 1: GTCTTTAGCTGCTGAGGAAATG und
   Primer 2: AGCAGAATTCTGCACATGACG) sowie
(b) eine Sequenz, die aus dem als "699" bezeichneten Locus entstammt
   (Probe: TGAACTGCTCCTTGGCAGGGATTT,
   Primer 1: TGCTCCCTGTCCCATCTG,
   Primer 2: AGACAGTATGCCTTTATTTCACCC).

Die Real-time PCR Reaktionen wurden im QuantiTect Master Mix (QIAGEN) entsprechend den Protokoll-Anweisungen durchgeführt.

Die Repräsentanz der Loci in der amplifizierten DNA wurde in Ct-Werten (Threshold-Cycles = Ct) gemessen. Der Ct-Wert ist der PCR-Zyklus in der Real-time PCR, an dem das Fluoreszenz-Signal zum ersten Mal meßbar wird. Durch den Ct-Wert kann somit die relative Häufigkeit einer Sequenz in der Probe ermittelt werden. Ist beispielsweise ein Ct-Wert um 1 Zyklus kleiner als in einer Vergleichsprobe, so entspricht dieser Wert einer ca. 2-fach höheren DNA-Ausgangsmenge im Testansatz gegenüber dem Kontrollansatz für die gemessene Sequenz.

Das Ergebnis von Beispiel 2 läßt sich wie folgt zusammenfassen: (1) Die Ausbeute aus den Reaktionen, bei denen die DNA durch einen 30 bis 65°C-Schritt aktiviert wurde, ist vergleichbar zur der Kontroll-Reaktion. (2) Auch die Repräsentanz der Sequenzen in der amplifizierten DNA ist nur etwas schlechter als bei der Kontrollreaktion, wenn die Aktivierung bei 60 oder 65°C durchgeführt wurde. Bei einer Aktivierung der DNA für die Strand-Displacement Reaktion unter 60°C wird die Repräsentanz der betrachteten Sequenzen schlechter.

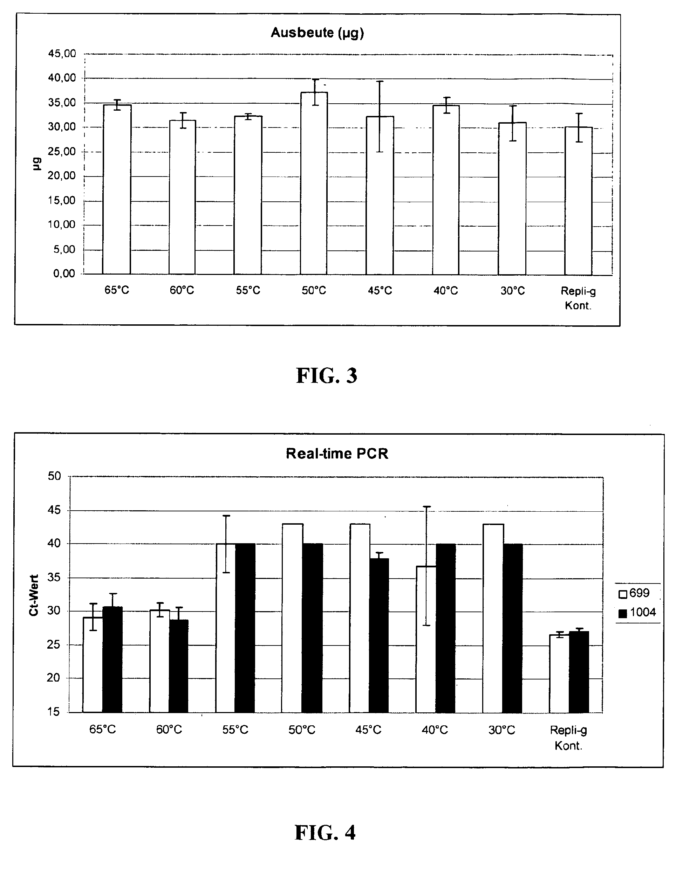

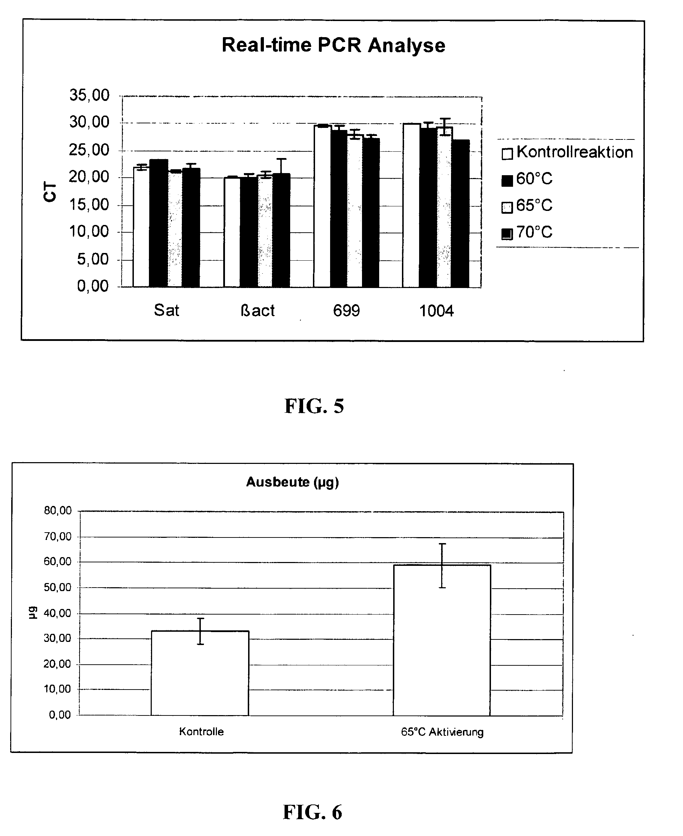

Die Ausbeute der Reaktionen aus Beispiel 2 ist in Fig. 3 graphisch dargestellt. Fig. 4 zeigt die Ct-Werte der Real-Time PCR aus Beispiel 2.

### Beispiel 3

Mit diesem Beispiel soll gezeigt werden, dass innerhalb bestimmter Temperaturgrenzen eine einfache Temperaturaktivierung vor der SDR die Qualität der in der SDR entstehenden DNA im Vergleich zu einer Reaktion nach dem Stand der Technik (Kontrollreaktion) nicht beeinträchtigt.

Erfindungsgemäße Reaktion: Die MDA-Reaktion wurde mit den REPLI-G Reagenzien (QIAGEN) durchgeführt. 0,5 µl Vollblut, stabilisiert durch EDTA, wurden mit 12,5 µl 4x Reaction Mix (dieser enthält Oligonukleotide mit einer Zufallssequenz, Reaktionspuffer und dNTPs) und Wasser auf ein Volumen von 39,5 µl aufgefüllt und durch verschiedene Temperaturen aktiviert (60, 65 und 70°C). Nach Abkühlen des Reaktionsansatzes auf Raumtemperatur (ca. 20-25 °C) wurde die DNA-Polymerase aus dem REPLI-G Kit zugegeben. Die Reaktion wurde für 6 h bei 30°C durchgeführt.

Kontroll-Reaktion nach dem Stand der Technik: Die MDA-Reaktion wurde mit den REPLI-G Reagenzien (QIAGEN) durchgeführt: 0,5 µl Vollblut, stabilisiert durch EDTA, wurden mit 2,5 µl PBS (REPLI-G Kit) versetzt. Anschließend wurden 3,5 µl des frisch angesetzten Denaturierungspuffers (360 mM KOH, 9 mM EDTA, 100 mM DTT) hinzu gegeben und für 10 min auf Eis inkubiert. Das Gemisch wurde nach der Inkubation mit Solution B (REPLI-G Kit) neutralisiert. Das Gemisch wurde mit 12,5 µl 4x Reaction Mix aus dem REPLI-G Kit (dieser enthält Oligonukleotide mit einer Zufallssequenz, Reaktionspuffer und dNTPs) und mit dest. Wasser auf ein Volumen von 49,5 µl aufgefüllt. Daraufhin wurden 0,5 µl der DNA-Polymerase aus dem REPLI-G Kit zugegeben. Die Reaktion wurde für 6 h bei 30°C durchgeführt.

Nach Ablauf der Reaktion wurde die DNA-Konzentration mit PicoGreen nach Hersteller-Protokoll (Molecular Probes) gemessen. 10 ng der MDA-DNA wurden für eine Real-time PCR eingesetzt. 4 verschiedene Loci wurden auf ihre Repräsentanz im Amplifikat hin untersucht:
(a) eine Sequenz, die aus dem als "1004" bezeichneten Locus entstammt
   (Probe: TGATGGCATTACTGGCACTTTGAGTTTTAC,
   Primer 1: GTCTTTAGCTGCTGAGGAAATG,
   Primer 2: AGCAGAATTCTGCACATGACG) und
(b) eine Sequenz, die aus dem als "699" bezeichneten Locus entstammt
   (Probe: TGAACTGCTCCTTGGCAGGGATTT,
   Primer 1: TGCTCCCTGTCCCATCTG,
   Primer 2: AGACAGTATGCCTTTATTTCACCC).
c) eine Sequenz, die als Sat bezeichnet wurde
   (Primer-Sequenzen:
   Sät 1.1 TCTTTCCACTCCATTGCAT und
   Satl.2 GGAATGGAATCAACCCAA
(d) eine Sequenz aus dem β-Aktin Gen
   **Primer 1 GTCTCAAGTCAGTGTACAGG**
   **Primer 2 GTGATAGCATTGCTTTCGTG**

Die Real-time PCR Reaktionen wurden im QuantiTect Master Mix (QIAGEN) entsprechend den Protokoll-Anweisungen durchgeführt.

Die Repräsentanz der Loci in der amplifizierten DNA wurde in Ct-Werten (Threshold-Cycles = Ct) gemessen. Der Ct-Wert ist der PCR-Zyklus in der Real-time PCR, an dem das Fluoreszenz-Signal zum ersten Mal meßbar wird. Durch den Ct-Wert kann somit die relative Häufigkeit einer Sequenz in der Probe ermittelt werden. Ist beispielsweise ein Ct-Wert um 1 Zyklus kleiner als in einer Vergleichsprobe, so entspricht dieser Wert einer ca. 2-fach höheren DNA-Ausgangsmenge im Testansatz gegenüber dem Kontrollansatz für die gemessene Sequenz.

Das Ergebnis läßt sich wie folgt zusammenfassen. Die Repräsentanz der Sequenzen in der amplifizierten DNA ist bei dem erfindungsgemäßen Ansatz teilweise besser als bei der Kontrollreaktion, wenn die Aktivierung bei 60°C, 65°C oder 70°C durchgeführt wurde.

Fig. 5 zeigt die Ct-Werte der Real-Time PCR-Analyse von DNA aus der SDR-Reaktion von Beispiel 3.

### Beispiel 4

Dieses Beispiel dient dazu zu zeigen, dass durch einen einfachen Temperaturaktivierungsschritt eine SDR (hier eine Multiple Displacement Amplification, MDA), ausgehend von isolierter genomischer DNA, ermöglicht wird, die hinsichtlich DNA-Ausbeute und DNA-Qualität vergleichbar zu einer Reaktion nach dem Stand der Technik (Kontrollreaktion) ist.

Erfindungsgemäße Reaktion: Die MDA-Reaktion wurde mit den REPLI-G Reagenzien (QIAGEN) durchgeführt. 2,5 µl einer Lösung von genomischer DNA aus humanen Zellen (Konzentration: 4 ng/µl) wurden mit 12,5 µl 4x Reaction Mix (dieser enthält Oligonukleotide mit einer Zufallssequenz, Reaktionspuffer und dNTPs) und Wasser auf ein Volumen von 39,5 µl aufgefüllt und durch einen Inkubationsschritt bei 65°C aktiviert. Nach Abkühlen des Reaktionsansatzes auf Raumtemperatur (ca. 20-25 °C) wurde die DNA-Polymerase aus dem REPLI-G Kit zugegeben. Die Reaktion wurde für 6 h bei 30°C durchgeführt.

Kontroll-Reaktion nach dem Stand der Technik: Die MDA-Reaktion wurde mit den REPLI-G Reagenzien (QIAGEN) durchgeführt. 2,5 µl genomische DNA aus humanen Zellen (Konzentration: 4 ng/µl) wurden mit 2,5 µl frisch angesetztem Denaturierungspuffer (50 mM KOH, 1,25 mM EDTA) versetzt und für 3 min bei Raumtemperatur inkubiert. Das Gemisch wurde nach der Inkubation mit einer 1:10 Verdünnung der Solution B (REPLI-g Kit) neutralisiert. Das Gemisch wurde mit 12,5 µl 4x Reaction Mix aus dem REPLI-G Kit (dieser enthält Oligonukleotide mit einer Zufallssequenz, Reaktionspuffer und dNTPs) und dest. Wasser auf ein Volumen von 49,5 µl aufgefüllt. Daraufhin wurden 0,5 µl der DNA-Polymerase aus dem REPLI-G Kit zugegeben. Die Reaktion wurde für 6 h bei 30°C durchgeführt.

Nach Ablauf der Reaktion wurde die DNA-Konzentration mit PicoGreen nach Hersteller-Protokoll (Molecular Probes) gemessen. 10 ng der MDA-DNA wurde für eine Real-time PCR eingesetzt. 1 Locus wurde auf die Repräsentanz im Amplifikat hin untersucht:
(a) eine Sequenz, die aus dem als "1004" bezeichneten Locus entstammt
   (Probe: TGATGGCATTACTGGCACTTTGAGTTTTAC,
   Primer 1: GTCTTTAGCTGCTGAGGAAATG,
   Primer 2: AGCAGAATTCTGCACATGACG).

Die Real-time PCR Reaktionen wurden im QuantiTect Master Mix (QIAGEN) entsprechend den Protokoll-Anweisungen durchgeführt.

Die Repräsentanz der Loci in der amplifizierten DNA wurde in Ct-Werten (Threshold-Cycles = Ct) gemessen. Der Ct-Wert ist der PCR-Zyklus in der Real-time PCR, an dem das Fluoreszenz-Signal zum ersten Mal meßbar wird. Durch den Ct-Wert kann somit die relative Häufigkeit einer Sequenz in der Probe ermittelt werden. Ist beispielsweise ein Ct-Wert um 1 Zyklus kleiner als in einer Vergleichsprobe, so entspricht dieser Wert einer ca. 2-fach höheren DNA-Ausgangsmenge im Testansatz gegenüber dem Kontrollansatz für die gemessene Sequenz.

Das Ergebnis war, dass die Ausbeute aus den Reaktionen, bei denen die DNA durch einen 65°C-Schritt aktiviert wurde, fast doppelt so hoch war wie bei der Kontrolle nach dem Stand der Technik. Die Repräsentanz der Sequenzen in der amplifizierten DNA ist in der erfindungsgemäßen Probe und der Kontrollprobe vergleichbar.

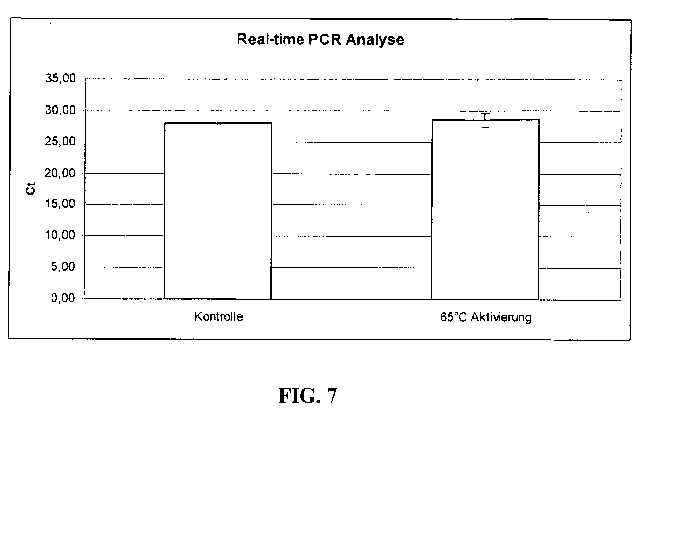

Die Ausbeute der Reaktionen aus Beispiel 4 ist in Fig. 6 graphisch dargestellt. Fig. 7 zeigt die Ct-Werte der Real-Time PCR aus Beispiel 4.

## Patentansprüche

1. Verfahren zur Aktivierung einer Nukleinsäure für eine Polymerase-Reaktion mit den Schritten:
(a) Erwärmen einer Nukleinsäure auf eine Temperatur von 50 bis 90°C,
(b) Abkühlen der Nukleinsäure auf eine Temperatur, bei der eine Polymerase keine wesentliche Abnahme der Aktivität zeigt, und
(c) Starten der Polymerase-Reaktion durch Zugabe einer hitzelabilen Polymerase zu der Nukleinsäure.

2. Verfahren zur Aktivierung einer Nukleinsäure für eine Polymerase-Reaktion mit dem Schritt:
(a) Erwärmen der Nukleinsäure zusammen mit einer hitzestabilen Polymerase auf eine Temperatur von 50 bis 90°C.

3. Verfahren nach Anspruch 1 oder 2, **dadurch gekennzeichnet, dass** die Polymerase-Reaktion eine Strand-Displacement-Reaktion ist.

4. Verfahren nach Anspruch 3, **dadurch gekennzeichnet, dass** die Strand-Displacement-Reaktion eine Multiple-Displacement-Reaktion ist.

5. Verfahren nach einem der vorhergehenden Ansprüche, **dadurch gekennzeichnet, daß** die Nukleinsäure eine DNA ist.

6. Verfahren nach einem der vorhergehenden Ansprüche, **dadurch gekennzeichnet, daß** die Nukleinsäure in Schritt (a) auf eine Temperatur von 55 bis 80°C, vorzugsweise 60 bis 70°C, insbesondere 65°C erwärmt wird.

7. Verfahren nach einem der Ansprüche 1, 3, 4, 5 oder 6, **dadurch gekennzeichnet, daß** in Schritt (b) die Nukleinsäure auf eine Temperatur von 4°C bis 45°C, bevorzugt 15°C bis 42°C, insbesondere 25 bis 37°C abgekühlt wird.

8. Verfahren nach einem der vorhergehenden Ansprüche, **dadurch gekennzeichnet, daß** die Nukleinsäure in gereinigter Form in einer wäßrigen Lösung vorliegt.

9. Verfahren nach einem der Ansprüche 1 bis 7, **dadurch gekennzeichnet, daß** die Nukleinsäure in einer Zelle vorliegt.
